# EUROPÄISCHE PATENTSCHRIFT

(11) **EP 1 003 716 B1**
(45) Veröffentlichungstag und Bekanntmachung des Hinweises auf die Patenterteilung: **18.02.2004**
(21) Anmeldenummer: 98945143.0
(22) Anmeldetag: 04.08.1998
(51) Int. Cl.: C07C 253/10, C07C 255/07

(54) **VERFAHREN ZUR HERSTELLUNG VON GEMISCHEN MONOOLEFINISCHER C 5-MONONITRILE DURCH KATALYTISCHE HYDROCYANIERUNG IN GEBENWART EINES KATALYSATORS, UMFASSEND WENIGSTENS EINEN METALLOCENPHOSPHOR (III)-NICKEL(0)-KOMPLEX**
METHOD FOR PRODUCING MIXTURES OF MONOOLEFINIC C 5-MONONITRILES BY CATALYTIC HYDROCYANATION IN THE PRESENCE OF A CATALYST CONTAINING AT LEAST ONE METALLOCENE PHOSPHOR (III)-NICKEL-(0) COMPLEX
PROCEDE DE PRODUCTION DE MELANGES DE MONONITRILES C 5-MONO-OLEFINIQUES PAR HYDROCYANURATION CATALYTIQUE EN PRESENCE D'UN CATALYSEUR CONTENANT AU MOINS UN COMPLEXE METALLOCENE-PHOSPHORE(III)-NICKEL(0)

(30) Priorität: 04.08.1997 DE 19733682
(43) Veröffentlichungstag der Anmeldung: 31.05.2000
(73) Patentinhaber: BASF AKTIENGESELLSCHAFT, 67056 Ludwigshafen (DE)
(72) Erfinder: FISCHER, Jakob, D-85414 Kirchdorf (DE); SIEGEL, Wolfgang, D-67117 Limburgerhof (DE)
(74) Vertreter: Kinzebach, Werner, Dr.
(86) Internationale Anmeldenummer: PCT/EP1998/004851
(87) Internationale Veröffentlichungsnummer: WO 1999/007671

(56) Entgegenhaltungen:
- EP-A- 0 274 401
- B. CORNELIS; W. A. HERRMANN: "Applied Homogeneous Catalysis with Organometallic Compounds; Vol. 1: Applications" 1996 , VCH , WEINHEIM NEW YORK BASEL CAMBRIDGE TOKYO XP002086595 in der Anmeldung erwähnt siehe Seite 465 - Seite 468 siehe insbesondere Seite 478 - Seite 480

## Beschreibung

Die vorliegende Erfindung betrifft ein Verfahren zur Herstellung von Gemischen monoolefinischer C₅-Mononitrile mit nichtkonjugierter C=C- und C≡N-Bindung durch katalytische Hydrocyanierung eines 1,3-Butadien-haltigen Kohlenwasserstoffgemisches.

Zur großtechnischen Herstellung von Polyamiden (Nylons) besteht weltweit ein großer Bedarf an α,ω-Alkylendiaminen, welche dabei als ein wichtiges Ausgangsprodukt dienen. α,ω-Alkylendiamine, wie z. B. das Hexamethylendiamin, werden fast ausschließlich durch Hydrierung der entsprechenden Dinitrile gewonnen. Fast alle großtechnischen Wege zur Herstellung von Hexamethylendiamin sind daher im wesentlichen Varianten der Herstellung des Adipodinitrils, von dem jährlich weltweit etwa 1,0 Mio. Tonnen produziert werden.

In K. Weissermel, H.-J. Arpe, Industrielle Organische Chemie, 4. Auflage, VCH Weinheim, S. 266 ff. sind vier prinzipiell unterschiedliche Routen zur Herstellung von Adipinsäuredinitril beschrieben:
1. die dehydratisierende Aminierung der Adipinsäure mit Ammoniak in der Flüssig- oder Gasphase über intermediär gebildetes Diamid;
2. die indirekte Hydrocyanierung des 1,3-Butadiens über die Zwischenstufe der 1,4-Dichlorbutene;
3. die Hydrodimerisierung von Acrylnitril in einem elektrochemischen Prozess; und
4. die direkte Hydrocyanierung von 1,3-Butadien mit Cyanwasserstoff.

Nach dem letztgenannten Verfahren erhält man in einer ersten Stufe durch Monoaddition ein Gemisch isomerer Pentennitrile, das in einer zweiten stufe zu vorwiegend 3- und 4-Pentennitril isomerisiert wird. Anschließend wird in einer dritten Stufe durch anti-Markownikow-Cyanwasserstoffaddition an 4-Pentennitril das Adipinsäuredinitril gebildet. Die Umsetzung erfolgt dabei in der Flüssigphase in einem Lösungsmittel, wie z. B. Tetrahydrofuran, bei einer Temperatur im Bereich von 30 - 150 °C und drucklos. Dabei werden als Katalysatoren Nickelkomplexe mit Phosphin- bzw. Phosphit-Liganden und Metallsalz-Promotoren verwendet. An Metallocene gebundene komplexe Phosphinliganden zur Stabilisierung des Nickels werden in dem oben genannten Review nicht beschrieben.

In "Applied Homogeneous Catalysis with Organometalic Compounds", Bd. 1, VCH Weinheim, S. 465 ff. wird allgemein die heterogen und homogen katalysierte Addition von Cyanwasserstoff an Olefine beschrieben. Dabei werden vor allem Katalysatoren auf Basis von nicht-Metallocen-gebundenen Phosphin- und Phosphit-Komplexen des Nickels und Palladiums verwendet, die eine hohe Produktselektivität, verbesserte Umsätze und verringerte Reaktionszeiten ermöglichen. Zur Herstellung von Adipinsäuredinitril durch Hydrocyanierung von Butadien werden vorwiegend Nickel(0)-Phosphitkatalysatoren, ggf. in Gegenwart einer Lewis-Säure als Promotor verwendet. Allgemein läßt sich die Reaktion in die drei Schritte gliedern: 1. Synthese von Mononitrilen durch Hydrocyanierung von 1,3-Butadien; 2. Isomerisierung; 3. Synthese von Dinitrilen. Bei der Bildung des Monoadditionsproduktes erhält man ein Isomerengemisch aus 3-Pentennitril und 2-Methyl-3-butennitril, wobei die Selektivität bezüglich des linearen 3-Pentennitrils in Abhängigkeit vom verwendeten Katalysator etwa 70 % oder weniger beträgt. Wird dieser erste Reaktionsschritt in Abwesenheit von Lewis-Säuren durchgeführt, so findet im allgemeinen keine Zweitaddition von Cyanwasserstoff statt und das erhaltene Produktgemisch kann einer Isomerisierung unter Verwendung der gleichen Katalysatorsysteme wie im ersten Reaktionsschritt, diesmal gegebenenfalls in Gegenwart einer Lewis-Säure, wie z. B. ZnCl₂, als Promotor unterzogen werden. Dabei findet zum einen Isomerisierung von 2-Methyl-3-butennitril zu 3-Pentennitril und zum anderen die Isomerisierung von 3-Pentennitril zu den verschiedenen n-Pentennitrilen statt. In dieser Publikation ist erwähnt, daß das thermodynamisch stabilste Isomer, das 2-Pentennitril, bei dem die C,N-Dreifachbindung mit der C,C-Doppelbindung in Konjugation steht, die Reaktion inhibiert, da es als Katalysatorgift wirkt. Die gewünschte Isomerisierung zu 4-Pentennitril wird nur dadurch ermöglicht, daß 3-Pentennitril wesentlich schneller zu 4-Pentennitril als zu 2-Pentennitril isomerisiert wird.

Übliche Katalysatoren für die Hydrocyanierung von 1,3-Butadien sind vor allem die bereits genannten Nickel(0)-Phosphitkatalysatoren mit nicht-komplexmodifizierten Phosphitliganden.

Die EP-A-0 274 401 beschreibt ein Verfahren zur Hydrocyanierung von reinem Butadien unter Verwendung eines Nickelkatalysators, der eine Mischung aus Phenyl- und m,p-Tolyl-Phosphitliganden aufweist.

C.A. Tolman et al. beschreiben in Organometallics 1984, 3, S. 33 f. eine katalytische Hydrocyanierung von Olefinen durch Nikkel(0)-Phosphit-Komplexe unter spezieller Berücksichtigung der Effekte von Lewis-Säuren auf die Cyanwasserstoffaddition.

In Advances in Catalysis, Band 33, 1985, Academic Press, Inc., S. 1 f. wird übersichtsartig die homogen Nickel-katalysierte Hydrocyanierung von Olefinen beschrieben. Dabei wird insbesondere auf mechanistische Aspekte bei der Monohydrocyanierung von Butadien zu isomeren Pentennitrilen, der Isomerisierung von 2-Methyl-3-butennitril zu 3-Pentennitril und der Zweitaddition von Cyanwasserstoff zur Herstellung von Adipodinitril eingegangen. Als Katalysatoren werden Nickel(0)-Komplexe, vorzugsweise mit Phosphitliganden, eingesetzt.

Übliche Phosphine, wie z. B. Triphenylphosphin oder 1,2-Bis(diphenylphosphino)ethan weisen bei der Hydrocyanierung von Olefinen keine oder nur eine geringe katalytische Aktivität auf.

Die WO 95/30680 beschreibt zweizähnige Phosphin-Chelatliganden, bei denen die Phosphingruppen an Arylreste gebunden sind, die über zwei ortho-ständige Brücken annelliert sind. Dabei umfasst die erste Brücke ein O- oder S-Atom und die zweite Brücke ein O-, S- oder substituiertes N-, Si- oder C-Atom. Von den beiden Phosphinliganden befinden sich je einer an jedem Arylrest in ortho-Position zu der ersten Brücke. Diese zweizähnigen Phosphinliganden eignen sich in Form ihrer Übergangsmetallkomplexe als Katalysatoren für die Hydroformylierung, Hydrierung, Polymerisation, Isomerisation, Carboxylierung, Kreuzkupplung, Metathese und die Hydrocyanierung.

In J. Chem. Soc., Chem. Commun., 1995, S. 2177 f. wird der Effekt des Bindungswinkels der zuvor genannten, zweizähnigen Phosphinliganden auf die Aktivität und Selektivität bei der Nickel-katalysierten Hydrocyanierung von Styrol beschrieben.

In keiner der zuvor genannten Veröffentlichungen wird ein Verfahren zur katalytischen Hydrocyanierung unter Einsatz ein- oder mehrzähnige Nickel(0)-Phosphor(III)komplexe beschrieben, bei denen die Phosphor(III)liganden ihrerseits kovalent an einen oder beide der Cyclopentadienylliganden eines Metallocens gebunden sind.

Die EP-A 564 406 und die EP-A 612 758 beschreiben Ferrocenyldiphosphine als Liganden für homogene Katalysatoren und die Verwendung dieser Katalysatoren für die enantioselektive Hydrierung. Bei diesen Liganden sind zwei Phosphingruppen in ortho-Stellung an den gleichen Cyclopentadienylliganden des Ferrocens gebunden, einer davon unmittelbar an den C₅-Ring, der andere über eine substituierte C₁-Alkylengruppe. Die Rhodium- und Iridiumkomplexe mit diesen Liganden eignen sich als homogene enantioselektive Katalysatoren zur Hydrierung von prochiralen Verbindungen mit Kohlenstoff- oder Kohlenstoff-/Heteroatomdoppelbindungen. Der Einsatz dieser Katalysatoren für die Hydrocyanierung wird nicht beschrieben.

Katalysatoren für die asymmetrische Addition von Cyanwasserstoff an Alkene auf Basis von Übergangsmetall(0)-Komplexen sind bereits bekannt. So beschreibt das Aust. J. Chem., 1982, 35, S. 2041 f. die Verwendung von [(+)-(diop)]₂Pd und [(+)-(diop)]₂Ni ((+)-(diop) = (+)-(2S,3S)-(2,3-Isopropylidendioxybutan-1,4-diyl)bis(diphenylphosphin)) als Katalysatoren in der enantioselektiven Hydrocyanierung.

Im J. Am. Chem. Soc. 1996, 118, S. 6325 f. wird das Verhältnis von elektronischer Asymmetrie der Liganden zu beobachteter Enantioselektivität bei der asymmetrischen Hydrocyanierung unter Verwendung von elektronisch unsymmetrischen Bis-3,4-diarylphosphonit-Liganden auf der Basis von α-D-Fructofuranosiden beschrieben.

Der vorliegenden Erfindung liegt die Aufgabe zugrunde, ein Verfahren für die Hydrocyanierung zur Verfügung zu stellen, wobei die eingesetzten Katalysatoren insbesondere bei der Hydrocyanierung von 1,3-Butadien-haltigen Kohlenwasserstoffgemischen zur Herstellung von Gemischen monoolefinischer C₅-Mononitrile mit nichtkonjugierter C=C- und C≡N-Bindung sowie bei der Erst- und Zweitaddition von Cyanwasserstoff zur Herstellung von Adipodinitril eine hohe Selektivität und eine gute katalytische Aktivität aufweisen sollen.

Überraschenderweise wurde nun gefunden, dass die Aufgabe durch ein Verfahren gelöst wird, wobei man Katalysatoren einsetzt, die wenigstens einen Metallocenphosphor(III)-Nickel(0)-Komplex umfassen.

Gegenstand der vorliegenden Erfindung ist somit ein Verfahren zur Herstellung von Gemischen monoolefinischer C₅-Mononitrile mit nichtkonjugierter C=C- und C≡N-Bindung durch katalytische Hydrocyanierung von 1,3-Butadien oder eines 1,3-Butadien-haltigen Kohlenwasserstoffgemisches, das dadurch gekennzeichnet ist, dass die Hydrocyanierung in Gegenwart eines Katalysators erfolgt, der wenigstens einen Metallocenphosphor(III)-Nickel(0)-Komplex umfaßt, welcher mindestens einen ein- oder zweizähnigen Metallocenphosphor(III)liganden der allgemeinen Formel I 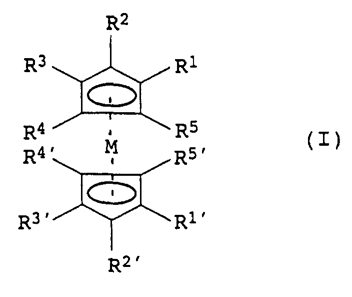 worin
- R¹: für einen Rest der Formel PL₂ steht, wobei die Reste L gleich oder verschieden sein können und für Alkyl, Cycloalkyl oder Aryl stehen,
- R^{1'}: für Wasserstoff, Alkyl oder einen Rest der Formel PL₂ steht, wobei die Reste L die zuvor angegebene Bedeutung besitzen,
- R², R^{2'}, R³, R^{3'}, R⁴, R^{4'}, R⁵, R^{5'}: unabhängig voneinander ausgewählt sind unter Wasserstoff, Cycloalkyl, Aryl oder Alkyl, welches durch ein Sauerstoffatom unterbrochen oder durch einen Rest der Formel NE¹E² substituiert sein kann, wobei E¹ und E² gleich oder verschieden sein können und für Alkyl, Cycloalkyl oder Aryl stehen, oder jeweils zwei der Substituenten R², R³, R⁴, R⁵ und/oder R^{2'}, R^{3'}, R^{4'}, R^{5'} in benachbarten Positionen zusammen mit dem sie verbindenden Teil des Cyclopentadienylrings für einen aromatischen oder nichtaromatischen 5- bis 7-gliedrigen Carbooder Heterocyclus stehen können, der 1, 2 oder 3 Heteroatome, ausgewählt unter O, N und S, aufweisen kann,
- M: für Fe, Co, Ni, Ru, Os, Rh, Mn, Cr oder V steht,
oder Salze und Mischungen davon,
umfaßt.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte C₁-C₈-Alkyl-, bevorzugter C₁-C₆-Alkyl- und besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl.

Bei der Cycloalkylgruppe handelt es sich vorzugsweise um eine C₅-C₇-Cycloalkylgruppe, wie Cyclopentyl, Cyclohexyl oder Cycloheptyl.

Wenn die Cycloalkylgruppe substituiert ist, weist sie vorzugsweise 1, 2, 3, 4 oder 5, insbesondere 1, 2 oder 3 Alkylreste als Substituenten auf.

Aryl steht vorzugsweise für Phenyl, Tolyl, Xylyl, Mesityl oder Naphthyl und insbesondere für Phenyl.

Substituierte Arylreste weisen als Substituenten, z. B. C₁-C₈-Alkyl, C₁-C₈-Alkoxy, Halogen, Trifluormethyl, Nitro oder Carboxyl auf. Dabei sind in der Regel 1, 2 oder 3 Substituenten bevorzugt.

Die Reste L der PL₂-Gruppen sind verschieden oder vorzugsweise gleich. Geeignete Reste L sind z. B. C₁-C₁₂-Alkyl und C₅-C₁₂-Cycloalkyl, welche durch eine, zwei oder drei der folgenden Gruppen: C₁-C₄-Alkyl, C₁-C₄-Alkoxy substituiert sein können. Weitere geeignete Reste L sind z. B. Aryl, welches durch eine, zwei oder drei der folgenden Gruppen: C₁-C₄-Alkyl, C₁-C₄-Alkoxy, Halogen, Trifluormethyl, Sulfo, Alkylsulfonyl, Carboxyl, Alkanoyloxy, Alkoxycarbonyl, Hydroxy, Nitro oder Cyano substituiert sein kann.

Vorzugsweise stehen die Reste L für Phenyl.

Wenn einer der Reste R², R^{2'}, R³, R^{3'}, R⁴, R^{4'}, R⁵ oder R^{5'} für Alkyl steht, welches durch ein Sauerstoffatom in Etherbindung unterbrochen ist, so können sie z. B. für 2-Methoxyethyl, 2-Ethoxyethyl, 2-Propoxyethyl, 2-Butoxyethyl, 2- oder 3-Methoxypropyl, 2-oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Butoxybutyl und vorzugsweise für 2-Methoxyethyl stehen.

Wenn einer der Reste R², R^{2'}, R³, R^{3'}, R⁴, R^{4'}, R⁵ oder R^{5'} für Alkyl steht, welches durch einen Rest der Formel NE¹E² substituiert ist, so können sie z. B. für N,N-Dimethyl-aminomethyl, N,N-Dimethyl-aminoethyl, N,N-Diethyl-aminomethyl, N,N-Diethyl-aminoethyl oder N,N-Dimethyl-aminopropyl stehen.

Wenn jeweils zwei der Substituenten R², R³, R⁴, R⁵ und/oder R^{2'}, R^{3'}, R^{4'}, R^{5'} in benachbarten Positionen zusammen mit dem sie verbindenden Teil des Cyclopentadienylrings für einen aromatischen oder nichtaromatischen, 5- bis 7-gliedrigen Carbo- oder Heterocyclus stehen, der zusätzlich 1, 2 oder 3 Heteroatome, ausgewählt unter O, N und S, aufweist, so können sie z. B. für Indenyl, Fluorenyl, Azulenyl etc. stehen.

Die beiden Cyclopentadienylringe des Metallocenphosphor(III)liganden der Formel I können in ekliptischer oder gestaffelter Konformation mit variierenden Konformationswinkeln vorliegen. Die Ebenen der Cyclopentadienylringe können z. B. in Abhängigkeit vom Zentralmetall, parallel oder gegeneinander geneigt sein. Die erfindungsgemäß eingesetzten Katalysatoren umfassen Metallocenphosphor(III)-Nickel(0)-Komplexe mit
a) Metallocenphosphor(III)liganden der Formel I, die nur einen Rest der Formel PL₂ aufweisen,
b) zweizähnige Metallocenphosphor(III)liganden der Formel I, die zwei Reste der Formel PL₂ aufweisen und bei denen die zuvor genannten strukturellen Begebenheiten des Liganden der Formel I eine zweifache Koordinierung eines Übergangsmetalls ermöglichen, und
c) zwei- oder mehrzähnige Metallocenphosphor(III)liganden der Formel I, bei denen ein Ligand über unterschiedliche Reste der Formel PL₂ unterschiedliche Übergangsmetalle koordiniert, wobei kettenartige Metallocenphosphor(III)-Nickel(0)-Komplexe, z. B. in Sandwich-Struktur, resultieren können.

Ein nullwertiges Übergangsmetall kann dabei einen, zwei, drei oder vier Liganden der Formel I und gegebenenfalls weitere Liganden, die im folgenden beschrieben werden, koordinieren.

Das Zentralmetall des Metallocenphosphor(III)liganden steht vorzugsweise für Fe.

Nach einer geeigneten Ausführungsform des erfindungsgemäßen Verfahrens werden Katalysatoren eingesetzt, worin
- R¹: für einen Rest der Formel PL₂ steht, wobei die Reste L für Phenyl stehen,
- R^{1'}: für Wasserstoff oder einen Rest der Formel PL₂ steht, wobei die Reste L unabhängig voneinander für Alkyl, Cycloalkyl, substituiertes Aryl und insbesondere für Phenyl stehen,
einer der Substituenten R², R^{2'}, R³, R^{3'}, R⁴, R^{4'}, R⁵ oder R^{5'}, vorzugsweise derjenige in α-Stellung zu R¹ oder R^{1'}, für Wasserstoff oder Alkyl steht, welches gegebenenfalls durch ein Sauerstoffatom unterbrochen oder durch einen Rest der Formel NE¹E² substituiert sein kann, wobei E¹ und E² gleich oder verschieden sein können und für Alkyl stehen, und die übrigen Substituenten R², R^{2'}, R³, R^{3'}, R⁴, R^{4'}, R⁵, R^{5'} unabhängig voneinander für Wasserstoff oder Methyl stehen.

R^{1'} steht dann vorzugsweise für Wasserstoff oder einen Rest der Formel PL₂, wobei die Reste L für Isopropyl, Cyclohexyl, Trifluormethyl-substituiertes Phenyl oder Phenyl stehen.

Die erfindungsgemäßen Katalysatoren können zusätzlich zu den zuvor beschriebenen Metallocenphosphinliganden der allgemeinen Formel I zusätzlich noch wenigstens einen weiteren Liganden, der ausgewählt ist unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃ sowie ein-, zwei- und mehrzähnigen Phosphin-, Phosphinit-, Phosphonit- und Phosphitliganden aufweisen, der ebenfalls ein-, zwei- oder mehrzähnig ist und an das nullwertige Übergangsmetall koordiniert.

Nach einer geeigneten Ausführungsform des erfindungsgemäßen Verfahrens werden Katalysatoren eingesetzt, die als Metallocenphosphor(III)ligand
1,1'-Bis(diphenylphosphino)ferrocen,
1,1'-Bis(dicyclohexylphosphino)ferrocen, oder
1,1'-Bis(diisopropylphosphino)ferrocen umfassen.

Nach einer möglichen Ausführungsform umfasst der erfindungsgemäß eingesetzte Katalysator einen chiralen Metallocenphosphinliganden der allgemeinen Formel I, so dass er sich zur enantioselektiven Katalyse eignet. Im allgemeinen wird bei der enantioselektiven Katalyse eine bereits chirale oder prochirale Verbindung unter Einsatz des erfindungsgemäßen Katalysators umgesetzt, wobei Produkte mit einem enantiomeren Überschuss oder vorzugsweise enantiomerenreine Produkte erhalten werden.

Zur Herstellung der erfindungsgemäßen Katalysatoren kann man mindestens einen Metallocenphosphinliganden der Formel I und Nickelpulver oder eine Nickelverbindung oder einen Nickelkomplex in einem inerten Lösungsmittel zur Reaktion bringen. Geeignete Nickelverbindungen sind dabei z. B. Verbindungen, in denen das Übergangsmetall eine Oxidationsstufe höher als 0 einnimmt, und die bei der Umsetzung mit dem Metallocenphosphor(III)liganden der Formel I, gegebenenfalls in Gegenwart eines geeigneten Reduktionsmittels, in situ reduziert werden. Dazu zählen z. B. die Halogenide, bevorzugt die Chloride, die Acetate und die Acetylacetonate der zuvor genannten Übergangsmetalle. Zur Herstellung von Nickel(0)-Komplexen wird bevorzugt NiCl₂ eingesetzt. Geeignete Reduktionsmittel sind z. B. Alkalimetalle, wie Na und K, und Aluminium sowie Trialkylaluminiumverbindungen.

Werden zur Herstellung der Metallocenphosphor(III)-Nickel(0)-Komplexe bereits Komplexverbindungen des Übergangsmetalls eingesetzt, so liegt in diesen das Übergangsmetall vorzugsweise bereits nullwertig vor. Bevorzugt werden zur Herstellung Komplexe mit Liganden eingesetzt, die den zuvor genannten, zusätzlichen Liganden der erfindungsgemäßen Komplexe entsprechen. In diesem Falle erfolgt die Herstellung durch teilweisen oder vollständigen Ligandenaustausch mit den zuvor beschriebenen Metallocenphosphor(III)-liganden der Formel I.

Nach einer geeigneten Ausführungsform des erfindungsgemäßen Verfahrens ist der Nickelkomplex Bis(1,5-cyclooctadien)nickel(0).

Geeignete inerte Lösungsmittel zur Herstellung der Metallocenphosphor(III)-Nickel(0)-Komplexe sind beispielsweise Aromaten, wie Benzol, Toluol, Ethylbenzol, Chlorbenzol, Ether, vorzugsweise Diethylether und Tetrahydrofuran, oder Halogenalkane, beispielsweise Dichlormethan, Chloroform, Dichlorethan und Trichlorethan. Die Temperatur liegt dabei in einem Bereich von -70 °C bis 150 °C, vorzugsweise von 0 °C bis 100 °C, besonders bevorzugt etwa bei Raumtemperatur.

Zur Herstellung von Gemischen monoolefinischer C₅-Mononitrile mit nichtkonjugierter C=C- und C≡N-Bindung kann ein 1,3-Butadien-haltiges Kohlenwasserstoffgemisch in Gegenwart eines der zuvor beschriebenen Katalysatoren umgesetzt werden. Vorzugsweise werden dabei Gemische mit einem hohen Anteil an linearen Pentennitrilen erhalten.

Zur Herstellung von Gemischen monoolefinischer C₅-Mononitrile, die z.B. 3-Pentennitril und 2-Methyl-3-butennitril enthalten und die als Zwischenprodukte für die Weiterverarbeitung zur Adipodinitril geeignet sind, kann man reines Butadien oder 1,3-Butadien-haltige Kohlenwasserstoffgemische einsetzen.

Wird zur Herstellung von monoolefinischen C₅-Mononitrilen nach dem erfindungsgemäßen Verfahren ein Kohlenwasserstoffgemisch eingesetzt, so weist dieses vorzugsweise einen 1,3-Butadien-Gehalt von mindestens 10 Vol.-%, bevorzugt mindestens 25 Vol.-%, insbesondere mindestens 40 Vol.-%, auf.

1,3-Butadien-haltige Kohlenwasserstoffgemische sind in großtechnischem Maßstab erhältlich. So fällt z.B. bei der Aufarbeitung von Erdöl durch Steamcracken von Naphtha ein als C₄-Schnitt bezeichnetes Kohlenwasserstoffgemisch mit einem hohen Gesamtolefinanteil an, wobei etwa 40 % auf 1,3-Butadien und der Rest auf Monoolefine und mehrfach ungesättigte Kohlenwasserstoffe sowie Alkane entfällt. Diese Ströme enthalten immer auch geringe Anteile von im allgemeinen bis zu 5 % an Alkinen, 1,2-Dienen und Vinylacetylen.

Reines 1,3-Butadien kann z. B. durch extraktive Destillation aus technisch erhältlichen Kohlenwasserstoffgemischen isoliert werden.

C₄-Schnitte werden gegebenenfalls von Alkinen, wie z. B. Propin oder Butin, von 1,2-Dienen, wie z. B. Propadien, und von Alkeninen, wie z. B. Vinylacetylen, im wesentlichen befreit. Ansonsten werden u.U. Produkte erhalten, bei denen eine C=C-Doppelbindung in Konjugation mit der C≡N-Bindung steht. Aus "Applied Homogeneous Catalysis with Organometalic Compounds", Bd. 1, VCH Weinheim, S. 479 ist bekannt, dass das bei der Isomerisierung von 2-Methyl-3-butennitril und 3-Pentennitril entstehende, konjugierte 2-Pentennitril als ein Reaktionsinhibitor für die Zweitaddition von Cyanwasserstoff zu Adipinsäuredinitril wirkt. Es wurde festgestellt, daß die oben genannten, bei der Hydrocyanierung eines nicht vorbehandelten C₄-Schnittes erhaltenen konjugierten Nitrile auch als Katalysatorgifte für den ersten Reaktionsschritt der Adipinsäureherstellung, die Monoaddition von Cyanwasserstoff, wirken.

Daher entfernt man gegebenenfalls aus dem Kohlenwasserstoffgemisch solche Komponenten teilweise oder vollständig, die bei katalytischer Hydrocyanierung Katalysatorgifte ergeben, insbesondere Alkine, 1,2-Diene und Gemische davon. Zur Entfernung dieser Komponenten wird der C₄-Schnitt vor der Addition von Cyanwasserstoff einer katalytischen Teilhydrierung unterzogen. Diese Teilhydrierung erfolgt in Gegenwart eines Hydrierungskatalysators, der befähigt ist, Alkine und 1,2-Diene selektiv neben anderen Dienen und Monoolefinen zu hydrieren.

Geeignete heterogene Katalysatorsysteme für die selektive Hydrierung umfassen im allgemeinen eine Übergangsmetallverbindung, z. B. auf einem inerten Träger. Geeignete anorganische Träger sind die hierfür üblichen Oxide, insbesondere Silicium- und Aluminiumoxide, Alumosilikate, Zeolithe, Carbide, Nitride etc. und deren Mischungen. Bevorzugt werden als Träger Al₂O₃, SiO₂ und deren Mischungen verwendet. Insbesondere handelt es sich bei den verwendeten heterogenen Katalysatoren um die in den US-A-4,587,369; US-A-4,704,492 und US-A-4,493,906 beschriebenen, auf die hier in vollem Umfang Bezug genommen wird. Weiterhin geeignete Katalysatorsysteme auf Cu-Basis werden von der Fa. Dow Chemical als KLP-Katalysator vertrieben.

Die Addition von Cyanwasserstoff an 1,3-Butadien oder ein 1,3-Butadien-haltiges Kohlenwasserstoffgemisch, z. B. einen vorbehandelten, teilhydrierten C₄-Schnitt, kann kontinuierlich, semikontinuierlich oder diskontinuierlich erfolgen.

Nach einer geeigneten Variante des erfindungsgemäßen Verfahrens erfolgt die Addition des Cyanwasserstoffs kontinuierlich. Geeignete Reaktoren für die kontinuierliche Umsetzung sind dem Fachmann bekannt und werden z. B. in Ullmanns Enzyklopädie der technischen Chemie, Bd. 1, 3. Aufl., 1951, S. 743 ff. beschrieben. Vorzugsweise wird für die kontinuierliche Variante des erfindungsgemäßen Verfahrens eine Rührkesselkaskade oder ein Rohrreaktor verwendet.

Gemäß einer bevorzugten Variante des erfindungsgemäßen Verfahrens erfolgt die Addition des Cyanwasserstoffs an 1,3-Butadien oder ein 1,3-Butadien-haltiges Kohlenwasserstoffgemisch semikontinuierlich.

Das semikontinuierliche Verfahren umfasst:
a) Befüllen eines Reaktors mit dem Kohlenwasserstoffgemisch, gegebenenfalls einem Teil des Cyanwasserstoffs und einem gegebenenfalls in situ erzeugten, erfindungsgemäßen Hydrocyanierungskatalysator sowie gegebenenfalls einem Lösungsmittel,
b) Umsetzung des Gemisches bei erhöhter Temperatur und erhöhtem Druck, wobei bei semikontinuierlicher Fahrweise Cyanwasserstoff nach Maßgabe seines Verbrauchs eingespeist wird,
c) Vervollständigung des Umsatzes durch Nachreagieren und anschließende Aufarbeitung.

Geeignete druckfeste Reaktoren sind dem Fachmann bekannt und werden z. B. in Ullmanns Enzyklopädie der technischen Chemie, Bd. 1, 3. Auflage, 1951, S. 769 ff. beschrieben. Im allgemeinen wird für das erfindungsgemäße Verfahren ein Autoklav verwendet, der gewünschtenfalls mit einer Rührvorrichtung und einer Innenauskleidung versehen sein kann. Für die obigen Schritte gilt vorzugsweise folgendes zu beachten:
Schritt a):
   Der druckfeste Reaktor wird vor Beginn der Reaktion mit dem teilhydrierten C₄-Schnitt, Cyanwasserstoff einem Hydrocyanierungskatalysator sowie ggf. einem Lösungsmittel befüllt. Geeignete Lösungsmittel sind dabei die zuvor bei der Herstellung der erfindungsgemäßen Katalysatoren genannten, bevorzugt aromatischen Kohlenwasserstoffe, wie Toluol und Xylol, oder Tetrahydrofuran.
Schritt b):
   Die Umsetzung des Gemisches erfolgt im allgemeinen bei erhöhter Temperatur und erhöhtem Druck. Dabei liegt die Reaktionstemperatur im allgemeinen in einem Bereich von etwa 0 bis 200°C, bevorzugt etwa 50 bis 150 °C· Der Druck liegt im allgemeinen in einem Bereich von etwa 1 bis 200 bar, bevorzugt etwa 1 bis 100 bar, insbesondere 1 bis 50 bar, insbesondere bevorzugt 1 bis 20 bar. Dabei wird während der Reaktion Cyanwasserstoff nach Maßgabe seines Verbrauchs eingespeist, wobei der Druck im Autoklaven im wesentlichen konstant bleibt. Die Reaktionszeit beträgt etwa 30 Minuten bis 5 Stunden.
Schritt c):
   Zur Vervollständigung des Umsatzes kann sich an die Reaktionszeit eine Nachreaktionszeit von 0 Minuten bis etwa 5 Stunden, bevorzugt etwa 1 Stunde bis 3,5 Stunden anschließen, in der kein Cyanwasserstoff mehr in den Autoklaven eingespeist wird. Die Temperatur wird in dieser Zeit im wesentlichen konstant auf der zuvor eingestellten Reaktionstemperatur belassen. Die Aufarbeitung erfolgt nach gängigen Verfahren und umfaßt die Abtrennung des nicht umgesetzten 1,3-Butadiens und des nicht umgesetzten Cyanwasserstoffs, z. B. durch Waschen oder Extrahieren und die destillative Aufarbeitung des übrigen Reaktionsgemisches zur Abtrennung der Wertprodukte und Rückgewinnung des noch aktiven Katalysators.

Gemäß einer weiteren geeigneten Variante des erfindungsgemäßen Verfahrens erfolgt die Addition des Cyanwasserstoffs an das 1,3-Butadien-haltige Kohlenwasserstoffgemisch diskontinuierlich. Dabei werden im wesentlichen die bei semikontinuierlichen Verfahren beschriebenen Reaktionsbedingungen eingehalten, wobei in Schritt b) kein zusätzlicher Cyanwasserstoff eingespeist, sondern dieser komplett vorgelegt wird.

Allgemein läßt sich die Herstellung von Adipinsäuredinitril aus einem Butadien-haltigen Gemisch durch Addition von 2 Moläquivalenten Cyanwasserstoff in drei Schritte gliedern:
1. Herstellung von C₅-Monoolefingemischen mit Nitrilfunktion.
2. Isomerisierung des in diesen Gemischen enthaltenen 2-Methyl-3-butennitrils zu 3-Pentennitril und Isomerisierung des so gebildeten und des in den Gemischen bereits aus Schritt 1 enthaltenen 3-Pentennitrils zu verschiedenen n-Pentennitrilen. Dabei soll ein möglichst hoher Anteil an 3-Pentennitril und ein möglichst geringer Anteil an konjugiertem und als Katalysatorgift wirksamen 2-Pentennitril und 2-Methyl-2-butennitril gebildet werden.
3. Herstellung von Adipinsäuredinitril durch Addition von Cyanwasserstoff an das in Schritt 2 gebildete 3-Pentennitril welches zuvor "in situ" zu 4-Pentennitril isomerisiert wird. Als Nebenprodukte treten dabei z. B. 2-Methyl-glutarodinitril aus der Markownikow-Addition von Cyanwasserstoff an 4-Pentennitril oder der anti-Markownikow-Addition von Cyanwasserstoff an 3-Pentennitril und Ethylsuccinodinitril aus der Markownikow-Addition von Cyanwasserstoff an 3-Pentennitril auf.

Vorteilhafterweise eignen sich die erfindungsgemäß eingesetzten Katalysatoren auf Basis von Metallocenphosphor(III)liganden auch für die Stellungs- und Doppelbindungsisomerisierung in Schritt 2 und die Zweitaddition von Cyanwasserstoff in Schritt 3.

Nach einer bevorzugten Ausführungsform des erfindungsgemäßen Verfahrens beträgt das bei der Monoaddition von Cyanwasserstoff an das 1,3-Butadien-haltige Kohlenwasserstoffgemisch erhaltene Mengenverhältnis von 3-Pentennitril zu 2-Methyl-3-butennitril mindestens 5:1, bevorzugt mindestens 10:1, insbesondere mindestens 20:1. Daher kann dann im allgemeinen auf eine Aufteilung des Reaktionsverfahrens zur Herstellung von Adipodinitril in die drei Schritte: Monoaddition von Cyanwasserstoff an ein 1,3-Butadien-haltiges Kohlenwasserstoffgemisch; Isomerisierung; Addition von Cyanwasserstoff an in situ gebildetes 4-Pentennitril; verzichtet werden und die Addition von 2 Moläquivalenten Cyanwasserstoff an ein 1,3-Butadien-haltiges Kohlenwasserstoffgemisch als einstufiges Verfahren ausgelegt werden. Gegenstand der vorliegenden Erfindung ist daher auch ein Verfahren zur Herstellung von Adipodinitril, das dadurch gekennzeichnet ist, dass man
I) ein wie zuvor beschrieben hergestelltes Gemisch von C₅-Mononitrilen gegebenenfalls nach weiterer Aufarbeitung oder Isomerisierung katalytisch hydrocyaniert, oder
II) ein 1,3-Butadien-haltiges Kohlenwasserstoffgemisch in einem einstufigen Verfahren, ohne Aufarbeitung und Isomerisierung von C₅-Mononitrilen, in Gegenwart eines Katalysators der Formel I katalytisch hydrocyaniert.

Vorteilhafterweise zeigen die erfindungsgemäß eingesetzten Katalysatoren nicht nur eine hohe Selektivität im Bezug auf die bei der Hydrocyanierung von 1,3-Butadien-haltigen Kohlenwasserstoffgemischen erhaltenen Monoadditionsprodukte, sondern sie können bei der Hydrocyanierung auch mit einem Überschuss an Cyanwasserstoff versetzt werden, ohne dass es zu einer merklichen Abscheidung von inaktiven Nickel(II)-Verbindungen, wie z. B. Nickel(II)-Cyanid, kommt. Im Gegensatz zu bekannten Hydrocyanierungskatalysatoren auf Basis nicht-komplexer Phosphin- und Phosphitliganden eignen sich die Katalysatoren der Formel I somit nicht nur für kontinuierliche Hydrocyanierungsverfahren, bei denen ein Cyanwasserstoffüberschuss im Reaktionsgemisch im allgemeinen wirkungsvoll vermieden werden kann, sondern auch für semikontinuierliche Verfahren und Batch-Verfahren, bei denen im allgemeinen ein starker Cyanwasserstoffüberschuss vorliegt. Somit weisen die erfindungsgemäß eingesetzten Katalysatoren und die auf ihnen basierenden Verfahren zur Hydrocyanierung im allgemeinen höhere Katalysatorrückführungsraten und längere Katalysatorstandzeiten auf als bekannte Verfahren. Dies ist neben einer besseren Wirtschaftlichkeit auch unter ökologischen Aspekten vorteilhaft, da das aus dem aktiven Katalysator mit Cyanwasserstoff gebildete Nickelcyanid stark giftig ist und unter hohen Kosten aufgearbeitet oder entsorgt werden muss.

Neben der Hydrocyanierung von 1,3-Butadien-haltigen Kohlenwasserstoffgemischen eignen sich die Katalysatoren der Formel I im allgemeinen für alle gängigen Hydrocyanierungsverfahren. Dabei sei insbesondere die Hydrocyanierung von Styrol und 3-Pentennitril genannt.

Die zuvor beschriebenen Katalysatoren können auch für die Isomerisierung von 2-Methyl-3-butennitril zu 3-Pentennitril eingesetzt werden. Katalysatoren, die chirale Metallocenphosphor(III)liganden der Formel I umfassen, eignen sich zur enantioselektiven Hydrocyanierung.

Die Erfindung wird anhand der folgenden, nicht einschränkenden Beispiele näher erläutert.

### Beispiele

### Beispiel 1 (erfindungsgemäß):

### Hydrocyanierung von 1,3-Butadien

In einem Glasautoklaven werden unter Argon bei Raumtemperatur 0,82 g Bis(1,5-cyclooctadien)nickel(0), 3,32 g 1,1'-Bis(diphenylphosphino)ferrocen und 10 ml Toluol miteinander vermengt, wobei sich der Reaktionsansatz sofort rot-braun färbt. Nach etwa 1 Stunde wird eine Mischung aus 16,2 g 1,3-Butadien in 40 g Toluol zugegeben. Der Glasautoklave wird fest verschlossen und die Reaktionsmischung auf 90 °C erhitzt, wobei sich ein Anfangsdruck von 3,4 bar einstellt. Über einen Zeitraum von 80 Minuten wird die Temperatur konstant bei 90 °C gehalten und eine Mischung aus 4,0 g frisch destillierter Blausäure in 30 ml Toluol kontinuierlich zudosiert. Danach ist der Druck auf 1,4 bar gefallen. Anschließend lässt man zur Vervollständigung der Reaktion noch 110 Minuten bei 90 °C nachreagieren. Zum Nachspülen des Reaktoraustrags wird Toluol verwendet. Der Verlauf der Reaktion wird über Druck- und Temperaturmessung verfolgt.

Bei einer anschließenden Cyanid-Bestimmung nach Volhard wird kein Cyanid detektiert, somit liegt ein praktisch vollständiger Cyanwasserstoffumsatz vor.

GC-Analytik (Säule: 30 m Stabil-Wachs, Temperaturprogramm: 5 Minuten isotherm bei 50 °C, anschließendes Aufheizen auf 240 °C mit einer Geschwindigkeit von 5 °C/min, Gaschromatograph: Hewlett Pakkard HP-5890) mit innerem Standard (Benzonitril): 75 % 3-Pentennitril und 2-Methyl-3-butennitril, bezogen auf eingesetzten Cyanwasserstoff.

### Verhältnis 3-Pentennitril : 2-Methyl-3-butennitril = 30:1.

### Beispiel 2 (erfindungsgemäß):

### Hydrocyanierung von 1,3-Butadien

In einem Glasautoklaven werden unter Argon bei Raumtemperatur 0,14 g Bis(1,5-cyclooctadien)nickel(0), 1,65 g 1,1'-Bis(diphenylphosphino)ferrocen und 10 ml Toluol miteinander vermengt, wobei sich der Reaktionsansatz sofort rot-braun färbt. Nach etwa 1 Stunde wird eine Mischung aus 16,2 g 1,3-Butadien in 40 g Toluol zugegeben. Der Glasautoklave wird fest verschlossen und die Reaktionsmischung auf 80 °C erhitzt, wobei sich ein Anfangsdruck von 3,4 bar einstellt. Über einen Zeitraum von 90 Minuten wird bei konstant 80 °C eine Mischung aus 4,0 g frisch destillierter Blausäure in 30 ml Toluol kontinuierlich zudosiert. Danach ist der Druck auf 2,0 bar gefallen. Anschließend lässt man zur Vervollständigung der Reaktion noch 60 Minuten bei 80 °C nachreagieren. Zum Nachspülen des Reaktoraustrags wird Toluol verwendet. Der Verlauf der Reaktion wird über Druck- und Temperaturmessung verfolgt.

Bei einer anschließenden Cyanid-Bestimmung nach Volhard werden 0,004 Gew.-%, bezogen auf 120,1 g, Cyanid ermittelt. Somit liegt der Cyanwasserstoffumsatz bei 99,5 %.

GC-Analytik (Säule: 30 m Stabil-Wachs, Temperaturprogramm: 5 Minuten isotherm bei 50 °C, danach Aufheizen mit 5 °C/min auf 240 °C, Gaschromatograph: Hewlett Packard HP 5890) mit innerem Standard (Benzonitril): 81,7 % 3-Pentennitril und 2-Methyl-3-butennitril, bezogen auf eingesetzten Cyanwasserstoff.

### Verhältnis 3-Pentennitril : 2-Methyl-3-butennitril = 30:1.

### Beispiel 3 (erfindungsgemäß):

### Hydrocyanierung von C₄-Schnitt

**Tabelle 1:**

| Zusammensetzung des C₄-Schnittes | |
|---|---|
| Verbindung | Vol.-% |
| 1,3-Butadien | 40,50 |
| cis-2-Buten | 2,65 |
| trans-2-Buten | 4,30 |
| Isobuten | 30,20 |
| 1-Buten | 14,30 |
| Isobutan | 1,10 |
| n-Butan | 2,90 |
| Propin | 0,50 |
| Kohlendioxid | 0,10 |
| Vinylacetylen | 0,35 |

In einem Glasautoklaven werden unter Argon bei Raumtemperatur 0,41 g Bis(1,5-cyclooctadien)nickel(0), 0,80 g 1,1'-Bis(diphenylphosphino)ferrocen und 10 ml Toluol miteinander vermengt, wobei sich der Reaktionsansatz sofort rot-braun färbt. Nach etwa 1 Stunde wird eine Mischung aus 39 g C₄-Schnitt mit einer Zusammensetzung gemäß Tabelle 1 in 50 g Toluol zugegeben. Der Glasautoklave wird fest verschlossen und die Reaktionsmischung auf 80 °C erhitzt, wobei sich ein Anfangsdruck von 3,9 bar einstellt. Über einen Zeitraum von 120 Minuten wird eine Mischung aus 4,0 g frisch destillierter Blausäure in 30 ml Toluol kontinuierlich zudosiert. Danach ist der Druck auf 2,5 bar gefallen. Anschließend lässt man zum Vervollständigen der Reaktion noch 120 Minuten bei 80 °C nachreagieren. Zum Nachspülen des Reaktionsaustrages wird Toluol verwendet. Der Verlauf der Reaktion wird über Druck- und Temperaturmessung verfolgt.

Bei einer anschließenden Cyanid-Bestimmung nach Volhard werden 0,01 % Cyanid, bezogen auf 134,1 g, detektiert. Somit liegt ein Cyanwasserstoffumsatz von 99,9 % vor.

GC-Analytik (Säule: 30 m Stabil-Wachs, Temperaturprogramm: 5 Minuten Isotherm bei 50 °C, danach Aufheizen mit einer Geschwindigkeit von 5 °C/min auf 240 °C, Gaschromatograph: Hewlett Packard HP 5890) mit internem Standard (Benzonitril): 84,7 % 3-Pentennitril und 2-Methyl-3-butennitril, bezogen auf eingesetzten Cyanwasserstoff.

### Verhältnis 3-Pentennitril : 2-Methyl-3-butennitril = 23,8:1.

### Beispiel 4 (Vergleich):

### Hydrocyanierung mit Katalysator auf Basis von Triphenylphosphin

In einem Mini-Glasautoklaven werden unter Argon bei Raumtemperatur 6,0 g Toluol, 0,1 g Bis(1,5-cyclooctadien)nickel(0) und 0,39 g Triphenylphosphin miteinander vermengt. Nach etwa 1 Stunde wird zuerst 2,0 g 1,3-Butadien und dann 1,0 g frisch destillierte Blausäure zugegeben. Der Glasautoklave wird fest verschlossen und die Reaktionsmischung auf 80 °C temperiert und unter Eigendruck belassen. Nach einer Reaktionszeit von 5 Stunden lässt man abkühlen und analysiert anschließend den Reaktionsaustrag.

GC-Analytik (Säule: 30 m Stabil-Wachs, Temperaturprogramm: 5 Minuten Isotherm bei 50 °C, danach Aufheizen mit einer Geschwindigkeit von 5 °C/min auf 240 °C, Gaschromatograph: Hewlett Packard HP 5890) mit innerem Standard (Benzonitril): 5,7 % 3-Pentennitril und 2-Butennitril, bezogen auf eingesetzten Cyanwasserstoff.

Verhältnis 3-Pentennitril : 2-Methyl-3-butennitril = 0,91:1.

## Patentansprüche

1. Verfahren zur Herstellung von Gemischen monoolefinischer C₅-Mononitrile mit nichtkonjugierter C=C- und C≡N-Bindung durch katalytische Hydrocyanierung von 1,3-Butadien oder eines 1,3-Butadien-haltigen Kohlenwasserstoffgemisches, **dadurch gekennzeichnet, dass** die Hydrocyanierung in Gegenwart eines Katalysators erfolgt, der wenigstens einen Metallocenphosphor(III)-Nickel(0)-Komplex umfaßt, welcher mindestens einen ein- oder zweizähnigen Metallocenphosphor(III)liganden der allgemeinen Formel I 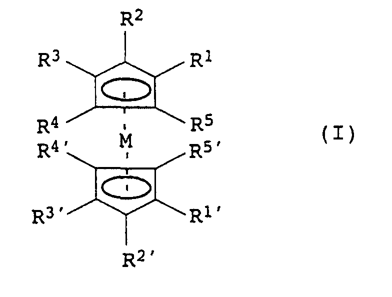 worin
R¹ für einen Rest der Formel PL₂ steht, wobei die Reste L gleich oder verschieden sein können und für Alkyl, Cycloalkyl oder Aryl stehen,
R^{1'} für Wasserstoff, Alkyl oder einen Rest der Formel PL₂ steht, wobei die Reste L die zuvor angegebene Bedeutung besitzen,
R², R^{2'}, R³, R^{3'}, R⁴, R^{4'}, R⁵, R^{5'} unabhängig voneinander ausgewählt sind unter Wasserstoff, Cycloalkyl, Aryl oder Alkyl, welches durch ein Sauerstoffatom unterbrochen oder durch einen Rest der Formel NE¹E² substituiert sein kann, wobei E¹ und E² gleich oder verschieden sein können und für Alkyl, Cycloalkyl oder Aryl stehen, oder jeweils zwei der Substituenten R², R³, R⁴, R⁵ und/oder R^{2'}, R^{3'}, R^{4'}, R^{5'} in benachbarten Positionen zusammen mit dem sie verbindenden Teil des Cyclopentadienylrings für einen aromatischen oder nichtaromatischen 5- bis 7-gliedrigen Carbooder Heterocyclus stehen können, der 1, 2 oder 3 Heteroatome, ausgewählt unter O, N und S, aufweisen kann,
M für Fe, Co, Ni, Ru, Os, Rh, Mn, Cr oder V steht,
oder Salze und Mischungen davon,
umfaßt.

2. Verfahren nach Anspruch 1, **dadurch gekennzeichnet, daß** man einen Katalysator der Formel I einsetzt, wobei M für Fe steht.

3. Verfahren nach einem der Ansprüche 1 oder 2, **dadurch gekennzeichnet, daß** man einen Katalysator der Formel I einsetzt, worin
R¹ für einen Rest der Formel PL₂ steht, wobei die Reste L für Phenyl stehen,
R^{1'} für Wasserstoff oder einen Rest der Formel PL₂ steht, wobei die Reste L für Phenyl stehen,
einer der Substituenten R², R^{2'}, R³, R^{3'}, R⁴, R^{4'}, R⁵ oder R^{5'}, vorzugsweise derjenige in α-Stellung zu R¹ oder R^{1'}, für Wasserstoff oder Alkyl steht, welches gegebenenfalls durch ein Sauerstoffatom unterbrochen oder durch einen Rest der Formel NE¹E² substituiert sein kann, wobei E¹ und E² gleich oder verschieden sein können und für Alkyl stehen, und die übrigen Substituenten R², R^{2'}, R³, R^{3'}, R⁴, R^{4'}, R⁵, R^{5'} unabhängig voneinander für Wasserstoff oder Alkyl stehen.

4. Verfahren nach einem der vorhergehenden Ansprüche, **dadurch gekennzeichnet, daß** man einen Katalysator einsetzt, der einen Liganden der Formel I und zusätzlich wenigstens einen weiteren Liganden, ausgewählt unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃ sowie ein-, zwei- und mehrzähnigen Phosphin-, Phosphinit-, Phosphonit- und Phosphitliganden aufweist.

5. Verfahren nach einem der vorhergehenden Ansprüche, **dadurch gekennzeichnet, daß** der Metallocenphosphor(III)ligand der Formel I ausgewählt ist unter
1,1'-Bis(diphenylphosphino)ferrocen,
1,1'-Bis(diisopropylphosphino)ferrocen oder
1,1'-Bis(dicyclohexylphosphino)ferrocen.

6. Verfahren nach einem der vorhergehenden Ansprüche, **dadurch gekennzeichnet, dass** man ein Kohlenwasserstoffgemisch mit einem 1,3-Butadiengehalt von mindestens 10 Vol.-%, bevorzugt mindestens 25 Vol.-%, insbesondere mindestens 40 Vol.-%, einsetzt.

7. Verfahren nach einem der vorhergehenden Ansprüche, **dadurch gekennzeichnet, dass** man als 1,3-Butadien-haltiges Kohlenwasserstoffgemisch einen C₄-Schnitt aus der Erdölverarbeitung einsetzt.

8. Verfahren nach einem der vorhergehenden Ansprüche, **dadurch gekennzeichnet, dass** die Hydrocyanierung semikontinuierlich erfolgt, wobei man
a) einen Reaktor mit dem Kohlenwasserstoffgemisch, einem, gegebenenfalls in situ erzeugten, Katalysator, gegebenenfalls einem Teil des Cyanwasserstoffs sowie gegebenenfalls einem Lösungsmittel befüllt,
b) das Gemisch bei erhöhter Temperatur und erhöhtem Druck umsetzt und Cyanwasserstoff nach Maßgabe seines Verbrauchs einspeist, und
c) gegebenenfalls zur Vervollständigung des Umsatzes nachreagieren lässt und anschließend aufarbeitet.

9. Verfahren nach einem der Ansprüche 1 bis 7, **dadurch gekennzeichnet, dass** die Hydrocyanierung diskontinuierlich erfolgt, wobei man in einem Verfahren nach Anspruch 8 die Gesamtmenge an Cyanwasserstoff in Stufe a) vorlegt.

10. Verfahren nach einem der vorhergehenden Ansprüche, **dadurch gekennzeichnet, dass** die Hydrocyanierung bei einer Temperatur von 0 bis 200 °C, bevorzugt 50 bis 150 °C, insbesondere 70 bis 120 °C, erfolgt.

11. Verfahren nach einem der vorhergehenden Ansprüche, **dadurch gekennzeichnet, dass** die Hydrocyanierung bei einem Druck von 1 bis 200 bar, bevorzugt 1 bis 100 bar, insbesondere 1 bis 50 bar und speziell 1 bis 16 bar erfolgt.

12. Verfahren nach einem der vorhergehenden Ansprüche, **dadurch gekennzeichnet, daß** der Reaktionsumsatz, bezogen auf Cyanwasserstoff, mindestens 95 %, bevorzugt mindestens 97 %, insbesondere bevorzugt mindestens 99 % beträgt.

13. Verfahren nach einem der vorhergehenden Ansprüche, **dadurch gekennzeichnet, daß** man ein Produktgemisch erhält, welches isomere Pentennitrile und Methylbutennitrile umfasst.

14. Verfahren nach Anspruch 13, **dadurch gekennzeichnet, dass** das Mengenverhältnis von 3-Pentennitril zu 2-Methyl-3-butennitril mindestens 5:1, bevorzugt mindestens 10:1, insbesondere mindestens 20:1, beträgt.

15. Verfahren nach einem der vorhergehenden Ansprüche, **dadurch gekennzeichnet, dass** der Katalysator zusätzlich zur Hydrocyanierung des 1,3-Butadiens auch zur Stellungs- und Doppelbindungsisomerisierung der monoolefinischen C₅-Mononitrile eingesetzt wird.

16. Verfahren zur Herstellung von Adipodinitril, **dadurch gekennzeichnet, daß** man ein Gemisch von C₅-Mononitrilen mit dem verfahren gemäß den Ansprüchen 1-14 herstellt und dieses gegebenenfalls nach weiterer Aufarbeitung und/oder Isomerisierung katalytisch hydrocyaniert.

17. Verfahren nach Anspruch 16, **dadurch gekennzeichnet, dass** die Hydrocyanierung des 1,3-Butadiens oder des 1,3-Butadien-haltigen Kohlenwasserstoffgemisches zur Herstellung von Adipodinitril einstufig, ohne separate Aufarbeitung und Isomerisierung von C₅-Mononitrilen, erfolgt.

18. Verwendung von Katalysatoren, umfassend einen Liganden der Formel I, gemäss Anspruch 1 zur Hydrocyanierung von Olefinen.

19. Verwendung von Katalysatoren, umfassend einen Liganden der Formel I gemäss Anspruch 1 , zur Hydrocyanierung und/oder Stellungs- und Doppelbindungsisomerisierung monoolefinischer C₅- Mononitrile.

## Claims

1. A process for preparing mixtures of monoolefinic C₅ mononitriles with nonconjugated C=C and C≡N bonds by catalytic hydrocyanation of 1,3-butadiene or a 1,3-butadiene-containing hydrocarbon mixture, wherein the hydrocyanation takes place in the presence of a catalyst which comprises at least one metallocene-phosphorus(III)-nickel(0) complex which comprises at least one monodentate or bidentate metallocene-phosphorus(III) ligand of the formula I 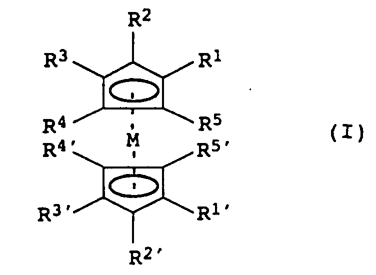 where
R¹ is a radical of the formula PL₂ where the L radicals can be identical or different and are alkyl, cycloalkyl or aryl,
R^{1'} is hydrogen, alkyl or a radical of the formula PL₂ where the L radicals have the abovementioned meaning,
R², R^{2'}, R³, R^{3'}, R⁴, R^{4'}, R⁵, R^{5'} are, independently of one another, selected from hydrogen, cycloalkyl, aryl or alkyl, which can be interrupted by an oxygen atom or substituted by a radical of the formula NE¹E² where E¹ and E² can be identical or different and are alkyl, cycloalkyl or aryl, or in each case two of the substituents R², R³, R⁴, R⁵ and/or R^{2'}, R^{3'}, R^{4'}, R^{5'} in adjacent positions can, together with the part of the cyclopentadienyl ring connecting them, be an aromatic or nonaromatic 5- to 7-membered carbocyclic or heterocyclic system which may have 1, 2 or 3 heteroatoms selected from O, N and S,
M is Fe, Co, Ni, Ru, Os, Rh, Mn, Cr or V,
or salts and mixtures thereof.

2. A process as claimed in claim 1, wherein a catalyst of the formula I where M is Fe is employed.

3. A process as claimed in either of claims 1 or 2, wherein a catalyst of the formula I where
R¹ is a radical of the formula PL₂ where the L radicals are phenyl,
R^{1'} is hydrogen or a radical of the formula PL₂ where the L radicals are phenyl,
one of the substituents R², R^{2'}, R³, R^{3'}, R⁴, R^{4'}, R⁵ or R^{5'}, preferably that in the position α to R¹ or R^{1'}, is hydrogen or alkyl which may be interrupted by an oxygen atom or substituted by a radical of the formula NE¹E² where E¹ and E² can be identical or different and are alkyl, and the other substituents R², R^{2'}, R³, R^{3'}, R⁴, R^{4'}, R⁵, R^{5'} are, independently of one another, hydrogen or alkyl, is employed.

4. A process as claimed in any of the preceding claims, wherein a catalyst which has a ligand of the formula I and in addition at least one other ligand selected from halides, amines, carboxylates, acetylacetonate, aryl- or alkylsulfonates, hydride, CO, olefins, dienes, cycloolefins, nitriles, N-containing heterocycles, aromatic and heteroaromatic systems, ethers, PF₃ and monodentate, bidentate and polydentate phosphine, phosphinite, phosphonite and phosphite ligands is employed.

5. A process as claimed in any of the preceding claims, wherein the metallocene-phosphorus(III) ligand of the formula I is selected from
1,1'-bis(diphenylphosphino)ferrocene,
1,1'-bis(diisopropylphosphino)ferrocene or
1,1'-bis(dicyclohexylphosphino)ferrocene.

6. A process as claimed in any of the preceding claims, wherein a hydrocarbon mixture with a 1,3-butadiene content of at least 10% by volume, preferably at least 25% by volume, in particular at least 40% by volume, is employed.

7. A process as claimed in any of the preceding claims, wherein a C₄ cut from petroleum processing is employed as 1,3-butadiene-containing hydrocarbon mixture.

8. A process as claimed in any of the preceding claims, wherein the hydrocyanation is carried out semicontinuously by
a) charging a reactor with the hydrocarbon mixture and a catalyst which has been generated in situ where appropriate, with or without part of the hydrogen cyanide and with or without a solvent,
b) reacting the mixture at elevated temperature under elevated pressure and feeding hydrogen cyanide in at the rate of its consumption and
c) completing the conversion by subsequent reaction, if required, followed by workup.

9. A process as claimed in any of claims 1 to 7, wherein the hydrocyanation is carried out batchwise with the total amount of hydrogen cyanide being present in stage a) in a process as claimed in claim 8.

10. A process as claimed in any of the preceding claims, wherein the hydrocyanation is carried out at from 0 to 200°C, preferably 50 to 150°C, in particular 70 to 120°C.

11. A process as claimed in any of the preceding claims, wherein the hydrocyanation is carried out under a pressure of from 1 to 200 bar, preferably 1 to 100 bar, in particular 1 to 50 bar and specifically 1 to 16 bar.

12. A process as claimed in any of the preceding claims, wherein the conversion of hydrogen cyanide in the reaction is at least 95%, preferably at least 97%, particularly preferably at least 99%.

13. A process as claimed in any of the preceding claims, wherein the resulting product mixture comprises isomeric pentenonitriles and methylbutenonitriles.

14. A process as claimed in claim 13, wherein the ratio of the amounts of 3-pentenonitrile to 2-methyl-3-butenonitrile is at least 5:1, preferably at least 10:1, in particular at least 20:1.

15. A process as claimed in any of the preceding claims, wherein the catalyst is employed, in addition to the hydrocyanation of 1,3-butadiene, also for positional and double-bond isomerization of the monoolefinic C₅ mononitriles.

16. A process for preparing adiponitrile, which comprises preparation of a mixture of C₅ mononitriles using the process as claimed in claims 1 - 14, and catalytic hydrocyanation of said mixture where appropriate after further workup and/or isomerization.

17. A process as claimed in claim 16, wherein the hydrocyanation of 1,3-butadiene or of the 1,3-butadiene-containing hydrocarbon mixture to prepare adiponitrile takes place in one stage, without separate workup and isomerization of C₅ mononitriles.

18. The use of catalysts comprising a ligand of the formula I as claimed in claim 1 for the hydrocyanation of olefins.

19. The use of catalysts comprising a ligand of the formula I as claimed in claim 1 for the hydrocyanation and/or positional and double-bond isomerization of monoolefinic C₅ mononitriles.

## Revendications

1. Procédé de préparation de mélanges de mononitriles en C₅ monooléfiniques possédant une liaison C=C- et C≡N- non conjuguée au moyen de l'hydrocyanation de 1,3-butadiène ou d'un mélange d'hydrocarbures contenant du 1,3-butadiène, **caractérisé en ce que** l'on réalise l'hydrocyanation en présence d'un catalyseur qui comprend au moins un complexe métallocène-phosphore(III)-nickel(0), lequel comprend au moins un ligand métallocène-phosphore(III) mono- ou bidenté de formule générale I 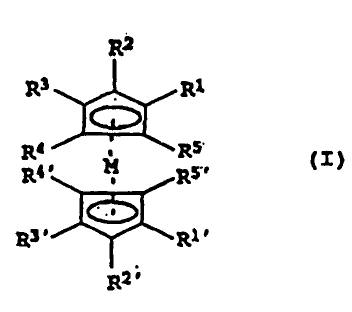 dans laquelle
R¹ représente un groupe de formule PL₂, les groupes L pouvant être identiques ou différents et représentant un groupe alkyle, cycloalkyle ou aryle,
R^{1'} représente un atome d'hydrogène, un groupe alkyle ou un groupe de formule PL₂, les groupes L ayant les significations indiquées ci-dessus,
R², R^{2'}, R³, R^{3'}, R⁴, R^{4'}, R⁵, R^{5'} sont choisis, indépendamment les uns des autres, parmi un atome d'hydrogène, un groupe cycloalkyle, aryle ou alkyle, lequel peut être interrompu par un atome d'oxygène ou substitué par un groupe de formule NE¹E², E¹ et E² pouvant être identiques ou différents et pouvant représenter un groupe alkyle, cycloalkyle ou aryle, ou bien dans chaque cas, deux des substituants R², R³, R⁴, R⁵ et/ou R^{2'}, R^{3'}, R^{4'}, R^{5'} dans des positions vicinales, conjointement avec la partie du cycle de cyclopentadiènyle les reliant, pouvant représenter un carbocycle ou un hétérocycle à 5 à 7 éléments aromatique ou non aromatique qui peut posséder 1, 2 ou 3 hétéroatomes, choisis parmi O, N et S,
M représente Fe, Co, Ni, Ru, Os, Rh, Mn, Cr ou V,
ou des sels et des mélanges de ceux-ci.

2. Procédé selon la revendication 1, **caractérisé en ce que** l'on utilise un catalyseur de formule I, dans laquelle M représente Fe.

3. Procédé selon la revendication 1 ou 2, **caractérisé en ce que** l'on utilise un catalyseur de formule I, dans laquelle
R¹ représente un groupe de formule PL₂, dans laquelle les groupes L représentent un groupe phényle,
R^{1'} représente un atome d'hydrogène ou un groupe de formule PL₂, dans laquelle les groupes L représentent un groupe phényle,
un des substituants R², R^{2'}, R³, R^{3'}, R⁴, R^{4'}, R⁵ ou R^{5'}, de préférence celui se trouvant en position α par rapport à R¹ ou R^{1'}, représente un atome d'hydrogène ou un groupe alkyle, lequel peut être éventuellement interrompu par un atome d'oxygène ou substitué par un groupe de formule NE¹E², E¹ et E² pouvant être identiques ou différents et pouvant représenter un groupe alkyle, et les autres substituants R², R^{2'}, R³, R^{3'}, R⁴, R^{4'}, R⁵, R^{5'} pouvant représenter, indépendamment l'un de l'autre, un atome d'hydrogène ou un groupe alkyle.

4. Procédé selon l'une quelconque des revendications précédentes, **caractérisé en ce que** l'on utilise un catalyseur qui possède un ligand de formule I et en outre, au moins un autre ligand, choisi parmi les halogénures, les amines, les carboxylates, les acétylacétonates, les aryl- ou les alkylsulfonates, les hydrures, CO, les oléfines, les diènes, les cyclooléfines, les nitriles, les hétérocycles contenant N, les aromates et les hétéroaromates, les éthers, PF₃ ainsi que les ligands mono-, bi- ou tridentés de phosphine, de phosphinite, de phosphonite et de phosphite.

5. Procédé selon l'une quelconque des revendications précédentes, **caractérisé en ce que** l'on choisit le ligand métallocène-phosphore(III) de formule I parmi
le 1,1'-bis(diphénylphosphino)ferrocène,
le 1,1'-bis(diisopropylphosphino)ferrocène ou
le 1,1'-bis(dicyclohexylphosphino)ferrocène.

6. Procédé selon l'une quelconque des revendications précédentes, **caractérisé en ce que** l'on utilise un mélange d'hydrocarbures présentant une teneur en 1,3-butadiène d'au moins 10% en volume, de préférence d'au moins 25% en volume, en particulier d'au moins 40% en volume.

7. Procédé selon l'une quelconque des revendications précédentes, **caractérisé en ce que** l'on utilise une coupe en C₄ issue d'un traitement de pétrole en tant qu'hydrocarbure contenant du 1,3-butadiène.

8. Procédé selon l'une quelconque des revendications précédentes, **caractérisé en ce que** l'on réalise l'hydrocyanation de manière semi-continue, dans lequel on
a) remplit un réacteur avec le mélange d'hydrocarbures, avec un catalyseur, préparé éventuellement in situ, éventuellement avec une partie de cyanure d'hydrogène ainsi qu'éventuellement avec un solvant,
b) fait réagir le mélange à une température élevée et une sous une pression élevée et on introduit le cyanure d'hydrogène en fonction de sa consommation, et,
c) on le soumet éventuellement une à réaction ultérieure afin de terminer la conversion puis, on procède à un retraitement.

9. Procédé selon l'une quelconque des revendications 1 à 7, **caractérisé en ce que** l'on réalise l'hydrocyanation de manière discontinue, en introduisant dans l'étape a, dans un procédé selon la revendication 8, la totalité du cyanure d'hydrogène.

10. Procédé selon l'une quelconque des revendications précédentes, **caractérisé en ce que** l'on réalise l'hydrocyanation à une température allant de 0°C à 200°C, de préférence de 50°C à 150°C, en particulier de 70°C à 120°C.

11. Procédé selon l'une quelconque des revendications précédentes, **caractérisé en ce que** l'on réalise l'hydrocyanation sous une pression allant de 1 bar à 200 bars, en particulier de 1 bar à 100 bars et tout particulièrement de 1 bar à 16 bars.

12. Procédé selon l'une quelconque des revendications précédentes, **caractérisé en ce que** le taux de conversion, exprimé par rapport au cyanure d'hydrogène, est d'au moins 95%, de préférence d'au moins 97%, en particulier d'au moins 99%.

13. Procédé selon l'une quelconque des revendications précédentes, **caractérisé en ce que** l'on obtient un mélange de produits contenant des pentènenitriles et des méthylbutènenitriles isomères.

14. Procédé selon la revendication 13, **caractérisé en ce que** le rapport de quantité du 3-pentènenitrile au 2-méthyl-3-butènenitrile est d'au moins 5:1, de préférence d'au moins 10:1, en particulier d'au moins 20:1.

15. Procédé selon l'une quelconque des revendications précédentes, **caractérisé en ce que** l'on utilise le catalyseur, outre pour l'hydrocyanation du 1,3-butadiène, pour l'isomérisation de position et à l'isomérisation de double liaison des mononitriles en C₅ monooléfiniques.

16. Procédé de préparation de l'adiponitrile, **caractérisé en ce que** l'on réalise l'hydrocyanation par voie catalytique, d'un mélange de mononitriles en C₅, préparé au moyen du procédé selon les revendications 1 à 14, éventuellement après un traitement supplémentaire et/ou une isomérisation.

17. Procédé selon la revendication 16, **caractérisé en ce que** l'on réalise l'hydrocyanation du 1,3-butadiène ou du mélange d'hydrocarbures contenant du 1,3-butadiène pour la préparation de l'adiponitrile, en une seule étape, sans traitement séparé et sans isomérisation de mononitriles en C₅.

18. Utilisation de catalyseurs contenant un ligand de formule I selon la revendication 1, pour l'hydrocyanation d'oléfines.

19. Utilisation de catalyseurs contenant un ligand de formule I selon la revendication 1, pour l'hydrocyanation et/ou pour l'isomérisation de position et l'isomérisation double liaison de mononitriles en C₅ monooléfiniques.
